# EUROPÄISCHE PATENTSCHRIFT

(11) **EP 0 799 090 B1**
(45) Veröffentlichungstag und Bekanntmachung des Hinweises auf die Patenterteilung: **19.05.1999**
(21) Anmeldenummer: 95942038.1
(22) Anmeldetag: 19.12.1995
(51) Int. Cl.: B01J 23/36, B01J 21/12, C07C 6/04, C07C 67/475

(54) **ALUMOSILIKAT-TRÄGER FÜR METATHESE KATALYSATOREN**
ALUMINOSILICATE CARRIER FOR METATHESIS CATALYSTS
SUPPORT EN SILICATE D'ALUMINIUM POUR CATALYSEURS DE METATHESE

(30) Priorität: 21.12.1994 DE 4445608
(43) Veröffentlichungstag der Anmeldung: 08.10.1997
(73) Patentinhaber: RWE-DEA AKTIENGESELLSCHAFT FÜR MINERALOEL UND CHEMIE, 22297 Hamburg (DE)
(72) Erfinder: NOWECK, Klaus, D-25541 Brunsbüttel (DE); HOFFMANN, Andreas, D-30629 Hannover (DE)
(74) Vertreter: Schupfner, Gerhard D.
(86) Internationale Anmeldenummer: DE9501846
(87) Internationale Veröffentlichungsnummer: WO9619287

(56) Entgegenhaltungen:
- DE-A- 4 107 056
- FR-A- 2 639 256

## Beschreibung

Die Erfindung betrifft auf hydrothermal behandelten Aluminiumsilikaten geträgerte Rheniumoxid-Katalysatoren ggf. mit Boroxid, Wolframoxid, Molybdänoxid oder Vanadiumoxid - Zusätzen für die Metathese von Olefinen und funktionalisierten Olefinen.

Die Metathese olefinischer Kohlenwasserstoffe dient in der Technik zur Herstellung von speziellen Olefinen, Di- und Polyenen und ungesättigter Polymeren. Polymere werden durch ringöffnende metathetische Polymerisation (ROMP) hergestellt. Metathesereaktionen können sowohl energiesparend bei Raumtemperatur als auch bei tieferen oder höheren Temperaturen durchgeführt werden.

Auch Olefine mit funktionellen Gruppen unterliegen der Metathesereaktion, sofern geeignete Katalysatoren zur Anwendung kommen. Von besonderer Bedeutung ist hierbei die Metathese ungesättigter Fettsäuremethylester. Durch die übergangsmetallkatalysierte Metathese dieser Ester, die in der Industrie durch Umesterung natürlicher Pflanzenöle und tierischer Fette mit niederen Alkoholen, insbesondere mit Methanol im 100000 t-Maßstab hergestellt werden, können wichtige Industriechemikalien für den Tensid- und Kunststoffsektor, sowie für die Produktion hochwertiger Schmierstoffe und einer Vielzahl chemisch-technischer Zwischenprodukte und Feinchemikalien gewonnen werden. Die prinzipielle Bedeutung der Metathese ungesättigter Fettsäureester für industrielle Anwendungen wurde in verschiedenen Publikationen beschrieben, vgl. z.B. M. Sibeijn et al.: 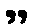 Technological and Economical Aspects of the Metathesis of Unsaturated Esters" in Journal of the American Oil Chemists' Society Vol. 7 1, Heft 6, S. 553 (1994).

Durch die sog. Homo-Metathese von ungesättigten Fettsäureestern werden langkettige Dicarbonsäureester erhalten: 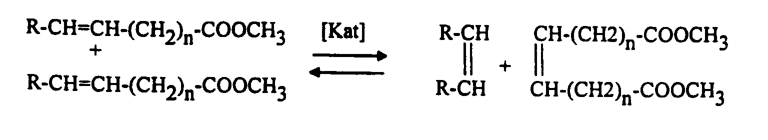 Durch die sog. Co-Metathese von ungesättigten Fettsäureestern mit Olefinen gemäß 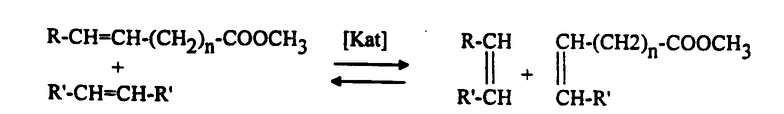 werden die Fettsäureester je nach verwendetem Olefin in ihrer Kettenlänge und/oder ihrer Struktur verändert. So können z.B. ungesättigte C₁₈-Fettsäuremethylester, die aus Raps-, Sonnenblumen- oder Sojaöl bzw. aus Talg hergestellt wurden, durch Co-Metathese mit petrochemischen C₄- bis C₆-Olefinen (Butene, Pentene, Hexene) in mittelkettige Fettsäureester des sog. Lauric-Bereichs mit Kettenlängen von insbesondere 12 bis 14 überführt werden.

Ester dieser Kettenlängen werden in der Industrie bisher aus tropischen Ölen (Kokos- und Palmkernöl) gewonnen; sie dienen zur Herstellung von Fettalkoholen für die Produktion von Tensiden.

Durch die Co-Metathese ungesättigter Fettsäureester mit Ethylen werden endständig ungesättigte Ester erhalten, die zur Herstellung von Dicarbonsäuren unterschiedlicher Kettenlänge und von organischen Zwischenprodukten und Feinchemikalien der verschiedensten Art dienen können. Durch Co-Metathese ungesättigter Fettsäureester mit verzweigten Olefinen werden verzweigte ungesättigte Fettsäureester erhalten, die Bedeutung für die Herstellung von Imprägniermitteln und speziellen Schmierstoffen in der Technik und Kosmetik haben.

Als Katalysatoren für die Metathesereaktionen werden bevorzugt Re₂0₇-Trägerkontakte eingesetzt, die mit metallorganischen Verbindungen wie Zinn- oder Bleialkylen der allgemeinen Formeln SnR₄ bzw. PbR₄ aktiviert werden. Ein typischer Katalysator ist hierbei Re₂0₇/γ-Al₂0₃, aktiviert mit Zinntetraalkylen. Neben γ-Al₂0₃ als Trägersubstanz für Re₂0₇ sind auch Aluminiumsilikat-Träger aus der Literatur bekannt.

In der FR-A-2 521 872 enthält der Träger vorzugsweise nur 10 bis 30 Gew.-% SiO₂. Als Aktivatoren dienen hier organische Bleiverbindungen der allgemeinen Formel PbR₄. In NL-A-84 03 051 wird die Metathese an SiO₂-reichen Trägerkatalysatoren durchgeführt. Die Re₂0₇/Al₂0₃-SiO₂-Katalysatoren enthalten im Träger 65 bis 90 Gew.-% SiO₂. Die Aktivatoren sind vorzugsweise Zinntetraethyl und -tetrabutyl. In der EP-B1-0 444 265 werden Aluminiumsilikate mit 40 Gew.-% SiO₂ als Träger verwandt. Eine Aktivierung erfolgt hier mit Aluminumalkylalkoholaten der allgemeinen Formel AlRₙR'ₘ (mit n+m=3, R=Alkyl und R'=Alkoholat) oder polymeren Alkylaluminoxanen.

Die Herstellung der Katalysatoren erfolgt durch Imprägnierung der Träger mit Lösungen von Ammoniumperrhenat, anschließender Trocknung und Calcinierung im Luftstrom bei erhöhten Temperaturen. Nach der EP-B1-0 444 264 wird eine Steigerung der katalytischen Aktivität dann erreicht, wenn man die Al₂0₃ - SiO₂-Träger nicht nur mit Amoniumperrhenat (NH₄Re0₄), sondern auch mit Borsäure (H₃B0₃) imprägniert und auf diese Weise Katalysatoren der Zusammensetzung B₂0₃ - Re₂0₇ / Al₂0₃ - SiO₂ herstellt. Nach Trocknung und Erhitzen entsteht der oxidische Katalysator. Die Aktivierung erfolgt hier mit zinnorganischen Verbindungen als Cocatalysator.

Allen Verfahren aus der Literatur ist das zentrale Problem gemeinsam, daß für die praktische Anwendung der Katalysatoren zur Metathese funktionalisierter Olefine, insbesondere von ungesättigten Carbonsäureestern, die Katalysatoren in hohen Konzentrationen eingesetzt werden müssen, um zu brauchbaren Umsätzen zu gelangen. Damit ist eine wirtschaftliche Nutzung der Metathesereaktion ungesättigter Carbonsäureester nicht möglich.

Der vorliegenden Erfindung liegt die Aufgabe zugrunde aktivere Katalysatoren für die Metathese zu entwickeln. Ziel ist es bei möglichst kleinem Rheniumgehalten - der Rheniumgehalt bestimmt die Preiswürdigkeit des Verfahrens - einen hohen Olefinumsatz zu erreichen. Vor allem sollten die Katalysatoren bei der Metathese von ungesättigten Fettsäureestern höhere Umsätze und höhere Ausbeuten ermöglichen.

Neben der hohen katalytischen Aktivität der erfindungsgemäßen Katalysatoren liegt ihr besonderer Vorteil darin, daß sie - nach jeweiliger thermischer Reaktivierung - vielfach wiederverwendet werden können. Dieser Vorteil ist für eine technische Anwendung von entscheidender Bedeutung. Diese Eigenschaft ist überraschend, da nach dem Stand der Technik über die Recycling - Fähigkeit von Rheniumoxid-Trägerkatalysatoren bei der Metathese ungesättigter Carbonsäureester nichts bekannt ist.

Erfindungsgemäß wird diese Aufgabe dadurch gelöst, daß man auf Aluminiumsilikaten geträgerte Rheniumoxid-Katalysatoren ggf. mit Boroxid-, Wolframoxid-, Molybdänoxid- oder Vanadiumoxid- Zusätzen verwendet, wobei der Aluminiumsilikat-Träger aus einer Silicium-Verbindung wie Kieselsäure, die als SiO₂-Lieferant dient, und einer Aluminium-Verbindung in wäßrigem Milieu hergestellt sind und das Aluminiumsilikat /Wasser Gemisch während und/oder nach, bevorzugt nach, der Reaktion der Silicium-Verbindung mit der Aluminium-Verbindung einer Temperaturbehandlung unterzogen wird. Diese Katalysatoren werden bevorzugt für die Metathese von Olefinen und funktionalisierten Olefinen insbesondere ungesättigter Carbonsäureester verwendet.

In der DE-C1-38 39 580 wird ein Verfahren zur Herstellung eines solchen hydrothermal behandelten Katalysatorträgers auf Basis eines Aluminiumsilikates mit 0,5 bis 50 Gew.-% SiO₂ und gegebenenfalls 0,5 bis 2 Gew.-% La₂0₃ durch Vermischen einer Aluminium-Verbindung mit einer Kieselsäureverbindung in wäßrigem Milieu gegebenenfalls unter gleichzeitigem oder anschließendem Zusatz einer löslichen Lanthanverbindung, Trocknen sowie gegebenenfalls Calcinieren des erhaltenen Produktes bis zu 24 Stunden bei einer Temperatur bis 11OO°C. beschrieben.

Die erfindungsgemäßen Aluminiumsilikate als Katalysator-Trägermaterial werden aus einem SiO₂-Lieferanten und einer Aluminium-Verbindung bevorzugt in wäßrigem Milieu hergestellt. Die Silicium-Verbindung als SiO₂ - Lieferant ist bevorzugt eine Kieselsäure und kann z.B. über Ionenaustauscher gereinigte Orthokieselsäure sein. Das Aluminiumalkoholat wird mit z.B. über Ionenaustauschern gereinigtem Wasser hydrolysiert. Dabei wird gleichzeitig oder anschließend die z.B. über Ionenaustauscher gereinigte Orthokieselsäure zugesetzt bzw. mit ihr vermischt. Als gegenüber Wasser und/oder Kieselsäure reaktive Aluminiumkomponente werden bevorzugt C2- bis C20- Aluminiumalkoholate verwendet. Die freigesetzten Alkohole werden nach der Reaktion aus der Reaktionsmischung entfernt.

Die hydrothermale Behandlung ist ein wesentlicher Teil des Verfahrens der Aluminiumsilikatherstellung für die erfindungsgemäßen Träger und besteht in einer Temperaturbehandlung während und/oder nach, bevorzugt nach der Reaktion der Kieselsäure-Verbindung mit der Aluminium-Verbindung, dabei wird das Aluminiumsilikat/Wassergemisch einer Temperatur von 90 bis 235 °C, vorzugsweise von 150 bis 200 °C, für einen Zeitraum von 0,5 bis 20 Stunden ,vorzugsweise aber für 1 bis 5 Stunden, unterworfen.

Die derart erhaltenen Katalysatorträger weisen eine hohe chemische Reinheit, eine homogene Verteilung des Al₂0₃ und des SiO₂, eine hohe spezifische Oberfläche (abhängig vom SiO₂-Gehalt) und eine ausgezeichnete Oberflächenstabilität, definierte Porenverteilungen, Porendurchmesser und hohe spezifische Aktivitäten auf.

**Tabelle 1**

| *hydrothermal gealtertes Aluminiumsilikate* | | | | |
|---|---|---|---|---|
| SiO₂-Gehalt [Gew.-%] | Oberfläche [m²/g] | Porenvolumen [ml/g] | Porenradius [Å] | Azidität [10⁻⁴ mmol n-Butylamin/m²] |
| 1,5 | 234 | 0,7 | 54 | 12,8 |
| 10 | 350 | 1,17 | 45 | 19,3 |
| 20 | 365 | 1,26 | 54 | 20,3 |
| 30 | 310 | 1,54 | 77 | 26,4 |
| 40 | 295 | 1,42 | 78 | 23,1 |
| 50 | 302 | 1,17 | 66 | 17,9 |

Die Porenvolumina und Porenradien wurden mit Hilfe der Quecksilberpenetrations-Porosimetrie (Autopore II 9220, Micromeritics-Porosimeter) bestimmt. Zur Bestimmung der spezifischen Oberfläche wurde eine BET-Adsorptionsisotherme (N₂) mit dem Gemini 2360 / 2375 der Firma Micromeritics aufgenommen.

Die verwendeten Al₂0₃ - SiO₂ -Träger weisen SiO₂ - Gehalte von 1.5 bis 50 Gew.-%, vorzugsweise 5 bis 30 Gew.-%, auf. Im Durchschnitt verändern sich durch die hydrothermale Behandlung Porenvolumen, Porenradius und Azidität, wie sich durch einen Vergleich mit Aluminiumsilikaten hergestellt ohne hydodrothermale Behandlung ergibt. Die gesteigerte Azidität korreliert mit der höheren katalytischen Aktivität.

**Tabelle 2**

| *nicht hydrothermal gealterte Aluminiumsilikate* | | | | |
|---|---|---|---|---|
| SiO₂-Gehalt [Gew.-%] | Oberfläche [m²/g] | Porenvolumen [ml/g] | Porenradius [Å] | Azidität [10⁻⁴ mmol n-Butylamin/m²] |
| 1,5 | 270 | 0,53 | 31 | 9,6 |
| 10 | 370 | 0,56 | 27 | 14,7 |
| 20 | 383 | 0,68 | 31 | 16,4 |
| 30 | 448 | 0,53 | 29 | 16,7 |
| 40 | 456 | 0,71 | 35 | 20,2 |
| 50 | 452 | 0,6 | 33 | 14,8 |

In einer weiteren Ausführungsform der Erfindung kann der Al₂O₃ - SiO₂ -Träger weiterhin 2 bis 10 Gew.-% besonders bevorzugt 3 bis 8 Gew.-% Boroxide, Molybdänoxide, Wolframoxide und Vanadiumoxide enthalten. Der Re₂0₇-Gehalt liegt zwischen 0.5 und 10 Gew.-%, vorzugsweise zwischen 1 und 5 Gew.-% bezogen auf den Träger.

Bei der Trägerung werden Rhenium und Bor, Wolfram, Molybdän oder Vanadium auf das Aluminiumsilikat aufgebracht. Diese Imprägnierung geschieht z.B. indem in Lösung befindliche Re-Verbindungen wie Ammoniumperrhenat und ggf. lösliche Bor Wolfram, Molybdän oder Vanadium -Verbindungen mit dem fertigen Aluminiumsilikat in Kontakt gebracht werden. Nach Trocknen und Erhitzen entsteht der oxidische Katalysator. Die Katalysatorbestandteile können entweder zusammen, z. B. in ein oder zwei Trankschritten, aufgebracht werden, oder nacheinander indem das Katalysatormaterial zweimal getrocknet und erhitzt wird, nämlich nach Aufbringen der ersten Komponenete und nach Aufbringen der zweiten Komponente.

In einer anderen Variante des Verfahrens zur Herstellung der erfindungsgemäßen Katalysatoren werden wasserlösliche Re- und ggf. Bor-, Wolfram-, Molybdän- oder Vanadium -Verbindungen entweder bei Hydrolyse der Aluminium-Verbindung oder bei Zugabe der Kieselsäure zugesetzt, danach wird der fertige Katalysator der erfindungsgemäßen Wärmebehandlung unterzogen.

Der optional eingesetzte Aktivator wird entweder zusammen mit den Edukten der Reaktion hinzugefügt oder aber vor Umsetzung mit den Olefinen dem fertig präparierten Re₂O₇-(B₂O₃, MoO₃, WO₃ oder V₂O₅) / Al₂O₃-SiO₂-Träger-Katalysator zugegeben bzw. getränkt.

Die Katalysatoren können als Pulver, Granulat, Extrudat oder als Wabenkörper eingesetzt werden. In Pulverform und Extrudatform werden die Katalysatoren vorzugsweise verwendet.

Bei der Metathese von Olefinkohlenwasserstoffen wird neben den erfindungsgemäßen Katalysatoren kein Aktivator benötigt. Bei der Metathese von funktionalisierten Olefinen oder bei der Co-Metathese von Olefinen und funktionalisierten Olefinen ist der Einsatz eines Aktivators jedoch notwendig. Funktionalisierte Olefine im Sinne der Erfindung sind ungesättigte Ester, Ether, Halogen- und Stickstoffverbindungen, Ketone sowie derivatisierte Alkohole und derivatisierte Carbonsäuren. Geeignete Aktivatoren sind zinnorganische Verbindungen, wobei man Zinntetraalkyl der Formel SnR₄, wobei R =Alkyl mit 1 bis 8 Kohlenstoffatomen ist, bevorzugt. Beispiele für R sind Methyl, Ethyl, lsopropyl und n-Butyl. Der Aktivator wird vorzugsweise in solchen Mengen eingesetzt, daß das molare Verhältnis von Re₂0₇: SnR₄ bei 5 : 1 bis 1 : 5 liegt. Im besonderen wird ein molares Verhältnis von 2 : 1 bis 1 : 2 bevorzugt.

Die erfindungsgemäßen Katalysatoren können nach Reaktivierung recycelt werden, indem die in der Katalyse benutzten Katalysatoren zunächst abgetrennt, getrocknet und dann bei erhöhter Temperatur von 300 bis 700 °C, vorzugsweise bei 350 bis 550 °C, thermisch behandelt werden. Die Reaktivierung kann mehrfach erfolgen und führt zu Katalysatoren mit nur geringfügig verminderter Metatheseaktivität.

Der erfindungsgemäße Katalysatorsystem ist besonders geeignet zur Metathese funktionalisierter Olefine, insbesondere von ungesättigten Carbonsäureestern. Die erfindungsgemäßen Katalysatoren können als Pulver, Granulat oder als Wabenkörper eingesetzt werden. Die Katalysatoren werden vorzugsweise in Pulverform verwendet.

Überraschenderweise wurde gefunden, daß Katalysatoren der Zusammensetzung (B₂0₃ oder WO₃ oder MoO₃ oder V₂O₅) - Re₂0₇ / Al₂0₃ - SiO₂ + Sn(n-C₄H₉)₄ sowie Re₂0₇ / Al₂0₃ - SiO₂ + Sn(n-C₄H₉)₄ mit jeweils hydrothermal behandelten Al₂0₃ - SiO₂ -Trägern deutlich wirksamer sind als die analogen Katalysatoren mit Al₂0₃ - SiO₂ - Trägern, die zwar nach dem gleichen Grundverfahren hergestellt wurden, jedoch keiner hydrothermalen Behandlung unterworfen worden waren.

Die besondere Bedeutung einer hydrothermalen Behandlung von Al₂0₃ - SiO₂-Träger für die Aktivität von Re₂0₇-Trägerkatalysatoren soll im folgenden an Vergleichsversuche abgehandelt werden.

Nach dem Stand der Technik (vgl. EP-B1-0444264) werden bei der Metathese von ungesättigten Carbonsäureestern, und zwar von 10-Undecensäuremethylester mit 4-Octen, in Gegenwart des Katalysators B₂0₃ - Re₂0₇ / Al₂0₃ - SiO₂, aktiviert mit Sn(n-C₄H₉)₄, die niedrigsten Katalysatorkonzentrationen mit einem molaren Verhältnis von Re₂0₇: SnR₄: Ester: 4-Octen = 1: 1,2: 800: 1600 angegeben, wobei ein Ester - Umsatz von 77 % erzielt wird.

Eigene Versuche zeigten, daß mit diesem Katalysator auch bei noch geringeren Konzentrationen gearbeitet werden kann. Bei einem molaren Verhältnis von Re₂0₇: Ester: Olefin = 1 / 3000 / 6000 sinkt allerdings der Umsatz auf 41 %, was für eine Durchführung der Reaktion in der Praxis zu gering ist (vgl. Beispiel 4). Mit dem erfindungsgemäßen B₂0₃ - Re₂0₇ / Al₂0₃ - SiO₂ -Katalysator (Beispiel 3) mit hydrothermal behandeltem Al₂0₃ - SiO₂ -Träger wird dagegen unter sonst identischen Bedingungen ein Umsatz von fast 70% erzielt.

Auch bei der Durchführung der genannten Metathesereaktion mit dem weniger aktiven B₂0₃-freien Re₂0₇-Katalysatoren (Re₂0₇ / Al₂0₃ - SiO₂ + SnBu₄) ist der aktivitätssteigernde Einfluß der hydrothermal behandelten Trägermaterialien signifikant , so steigt der Ester-Umsatz in Vergleichsversuchen - hier bei einem molaren Verhältnis von 1: 1000 - von 33 % (Beispiel 6) auf 62 % (Beispiel 5), wenn man die Al₂0₃ -SiO₂ -Träger bei sonst gleicher Zusammensetzung wechselt und zu hydrothermal behandeltem Material übergeht. Sofern eine B₂0₃-, WO₃ -, MoO₃ - oder V₂O₅- Modifizierung vorgenommen wird (Beispiel 7, 8 oder 9) wird eine weitere Aktivitätssteigerung auf einen Umsatz von über 75 % erzielt.

**Tabelle 3**

| *Co-Metathese von 10-Undecensäuremethylester mit 4-Octen, Beispiele 3 bis 10* | | | | | | | | | | |
|---|---|---|---|---|---|---|---|---|---|---|
| Beispiel | Re₂O₇ | SnBu₄ | Ester | 4-Octen | | hydrothermal Nachbeh. | C₁₁-Ester | C₁₄-Ester | C₂₀-Diester | Umsatz |
| | | [molares Verhältnis] | | | [Gew%] | | [Gew%] | [Gew%] | [Gew%] | [%] |
| **3** | 1 | 1,5 | 3000 | 6000 | 6,4 B₂O₃ | X | 30,6 | 62,5 | 6,9 | 66 |
| **4** | 1 | 1,5 | 3000 | 6000 | 6,4 B₂O₃ | - | 55,2 | 40 | 4,8 | 41 |
| **5** | 1 | 1,5 | 1000 | 2000 | - | X | 34,3 | 58,8 | 6,9 | 62 |
| **6** | 1 | 1,5 | 1000 | 2000 | - | - | 63,6 | 32,3 | 4,1 | 33 |
| **7** | 1 | 1,5 | 1000 | 2000 | 6,4 B₂O₃ | X | 17,0 | 75,2 | 7,8 | 81 |
| **8** | 1 | 1,5 | 1000 | 2000 | 6,4 WoO₃ | X | 22,0 | 71,1 | 6,9 | 76 |
| **9** | 1 | 1,5 | 1000 | 2000 | 6,4 MoO₃ | X | 18,9 | 75,5 | 5,4 | 78 |
| **10** | 1 | 1,5 | 1000 | 2000 | 6,4 V₂O₅ | X | 20,2 | 74,2 | 5,6 | 77 |

Beispiel 11 belegt die überraschend gefundene katatalytische Reaktivierung durch thermische Behandlung bereits eingesetzter erfindungsgemäßer Rheniumoxid-Trägerkatalysatoren.

### Beispiel 1

### Herstellung des Katalysators B₂0₃ - Re₂0₇ auf hydrothermal behandeltem Al₂0₃ - SiO₂ - Träger

10 g hydrothermal behandeltes Aluminiumsilikat (Al₂0₃ - SiO₂) mit 20 Gew.-% SiO₂ (siehe Tabelle 1) werden zunächst bei 550 °C in einem Strom getrockneter Luft 15 Stunden calciniert und anschließend unter Argon auf Raumtemperatur abgekühlt. Das so calcinierte Trägermaterial wird zusammen mit 0,55 g Ammoniumperrhenat (NH₄ReO₄) und 1,33 g Borsäure (H₃BO₃) sowie 90 ml 1,4-Dioxan und 10 ml destilliertem Wasser 12 Stunden unter Rückfluß erhitzt. Dann wird das Lösungsmittel abdestilliert und der zurückbleibende, nunmehr imprägnierte Al₂0₃ - SiO₂ - Träger wird bei 120 °C im Wasserstrahlvakuum getrocknet und schließlich - wie oben dargestellt - 12 Stunden bei 550 °C im Luftstrom calciniert. Der so hergestellte Katalysator B₂0₃ - Re₂0₇ / Al₂0₃ - SiO₂ enthält nominell 6.4 Gew.-% B₂0₃ und 5 Gew.-% Re₂0₇, jeweils bezogen auf den Al₂0₃ - SiO₂ - Träger.

### Beispiel 1a

Gemäß einem weiteren gegenüber 1 bevorzugtem Beispiel zur Imprägnierung des hydrothermal gealterten Aluminiumsilikat -Trägers werden 1,24 g NH₄ReO₄ und 2,58 g H₃BO₃ bei 60 °C in 25 g Wasser aufgelöst. Diese Lösung wird mittels eines Handzerstäubers unter intensiver Vermischung auf 20 g hydrothermal gealtertes Aluminiumsilikat (siehe Tabelle 1), z.B. Siral HT2O-Träger (Produkt der Condea Chemie GmbH, vorcalciniert bei 550°C), aufgebracht. Der so imprägnierte Katalysator wird ohne weitere Trocknung bei 550°C aktiviert und in die Metathese eingesetzt. Der Kontakt enthält in diesem Beispiel nominell 5,0 Gew.-% Re₂0₇ ; 6,4 Gew.-% B₂O₃ und 18 Gew.-% SiO₂. Die Differenz zu 100 % besteht aus Al₂0₃.

### Beispiel 2

### Herstellung des Katalysators Re₂0₇ auf einem hydrothermal behandeltem Al₂0₃ - SiO₂ -Träger

Die Katalysator-Herstellung von Beispiel 1 wird wiederholt, jedoch wird auf den Einsatz von Borsäure verzichtet. Es resultiert der Katalysator Re₂0₇ / Al₂O₃ -SiO₂ (Al₂O₃-SiO₂ hydrothermal behandelt) mit 5 Gew.-% Re₂O₇ bezogen auf den Al₂O₃ - SiO₂-Träger, der 20 Gew.-% SiO₂ enthält.

### Beispiel 3

### Co-Metathese von 10-Undecensäuremethylester mit 4-Octen

Katalysator: B₂0₃ - Re₂0₇ / Al₂0₃ - SiO₂ (Al₂O₃-SiO₂ hydrothermal behandelt, 20 Gew.-% SiO₂), aktiviert mit Sn(n-C₄H₉)₄

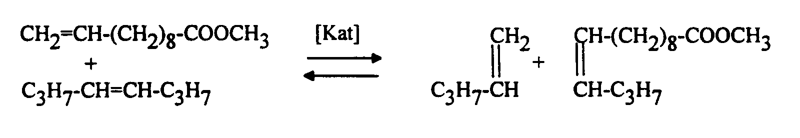

In einem ausgeheizten und mit Argon gefüllten 50 ml Glasautoklav werden 0,12 g des in Beispiel 1 dargestellten Katalysators (diese Katalysatormenge enthält 0,012 mmol Re₂0₇), 0,19 ml einer 0,1 molaren Zinntetrabutyl-Lösung in Cyclohexan (0,019 mmol Sn(n-C₄H₉)₄) sowie 7,36 g 10-Undecensäuremethylester (37 mmol) und 8,34 g 4-Octen (74 mmol) bei Raumtemperatur mit einem Magnetkernrührer gerührt.
Das molare Verhältnis der Reaktionskomponenten betrug: Re₂0₇ : SnBu₄: Ester: 4-Octen = 1 : 1,5 : 3000 : 6000
Nach 3 Stunden wird der pulverförmige Katalysator durch Zentrifugieren abgetrennt. Der Reaktionslösung wird eine Probe entnommen, die zur Zerstörung etwaiger Katalysatorreste mit einigen Tropfen Ethanol versetzt wird. Anschließend wird die Probe gaschromatographisch analysiert.
Gemäß obiger Reaktionsgleichung enthält die Reaktionslösung neben den Edukten nun auch 1-Penten und 10-Tetradecensäuremethylester sowie geringe Mengen an 10-Eicosen-1,20-dicarbonsäuredimethylester, der gemäß 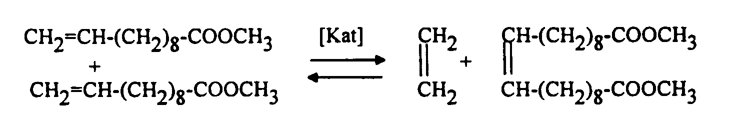 durch Selbstmetathese des eingesetzten 10-Undecensäuremethylesters gebildet wurde. Die Zusammensetzung der Ester im Reaktionsgemisch betrug 30,6 Gew.-% 10-Undecensäuremethylester, 62,5 Gew.-% 10-Tetradecensäuremethylester und 6,9 Gew.-% C₂₀-Dicarbonsäuredimethylester. Dieses entspricht einem Umsatz des verwendeten 10-Undecensäuremethylesters von 66 %.

### Beispiel 4 (Vergleichsbeispiel)

### Co-Metathese von 10-Undecensäuremethylester mit 4-Octen

Katalysator: B₂0₃ - Re₂0₇ / Al₂0₃ - SiO₂ (20 Gew.-% SiO₂), aktiviert mit Sn(n-C₄H₉)₄ Der Versuch von Beispiel 3 wird wiederholt, jedoch wird jetzt ein Katalysator eingesetzt, zu dessen Herstellung gemäß Beispiel 1 ein Aluminiumsilikat mit 20 Gew.-% SiO₂ verwendet wurde, bei dessen Präparation nach DE-C1-38 39 580 auf die hydrothermale Behandlung, d.h. auf die thermische Nachbehandlung verzichtet wurde.
Bei gleichen Mengenansätzen und Reaktionsbedingungen wie in Beispiel 3 sinkt jetzt der Umsatz des 10-Undecensäuremethylesters auf 41 %; die Esterzusammensetzung beträgt: 55,2 Gew.-% C₁₁-Ester, 40,0 Gew.-% C₁₄-Ester und 4,8 Gew.-% C₂₀-Diester.

### Beispiel 5

### Co-Metathese von 10-Undecensäuremethylester mit 4-Octen

Katalysator: Re₂0₇ / Al₂0₃ - SiO₂ (Al₂O₃-SiO₂ hydrothermal behandelt, 20 Gew.-% SiO₂), aktiviert mit Sn(n-C₄H₉)₄
Der Versuch von Beispiel 3 wird wiederholt, jedoch wird jetzt der nach Beispiel 2 hergestellte B₂0₃-freie Katalysator eingesetzt und ein molares Verhältnis der Reaktionkomponenten Re₂0₇: SnBu₄: Ester: 4-Octen = 1: 1,5: 1000: 2000 angewendet. Nach 3 Stunden bei Raumtemperatur wird ein Umsatz des C₁₁-Esters von 62 % festgestellt und die Ester-Zusammensetzung beträgt: 34,3 Gew.-% C₁₁Ester, 58,8 Gew.-% C₁₄-Ester und 6,9 Gew.-% C₂₀-Diester.

### Beispiel 6 (Vergleichsbeispiel)

### Co-Metathese von 10-Undecensäuremethylester mit 4-Octen Katalysator: Re₂0₇ / Al₂0₃ - SiO₂, aktiviert mit Sn(n-C₄H₉)₄

Der Versuch von Beispiel 5 wird wiederholt, jedoch wird jetzt ein Katalysator eingesetzt, zu dessen Herstellung gemäß Beispiel 2 ein Aluminiumsilikat mit 20 Gew.-% SiO₂ verwendet wurde, bei dessen Präparation nach DE-C1- 38 39 580 auf die hydrothermale Behandlung verzichtet wurde.
Bei gleichen Mengenansätzen und Reaktionsbedingungen wie in Beispiel 5 sinkt der Umsatz des C₁₁-Esters auf 33 %, die Esterzusammensetzung beträgt 63,6 Gew.-% C₁₁-Ester, 32,3 Gew.-% C₁₄-Ester und 4,1 Gew.-% C₂₀-Diester.

### Beispiel 7

### Co-Metathese von 10-Undecensäuremethylester mit 4-Octen

Katalysator: B₂0₃ - Re₂O₇/Al₂0₃ - SiO₂ (Al₂O₃-SiO₂ hydrothermal behandelt, 20 Gew.-% SiO₂), aktiviert mit Sn(n-C₄H₉)₄
Unter den Bedingungen von Beispiel 5, d.h. molares Verhältnis Re₂O₇: SnBu₄: Ester: 4-Octen = 1 : 1,5 : 1000 : 2000, jedoch mit dem B₂0₃-haltigen Katalysator aus Beispiel 1 steigt der Umsatz nach 3 Stunden bei Raumtemperatur auf 81 % und die Esterzusammensetzung betrug 17,0 Gew.-% C₁₁-Ester, 75,2 Gew.-% C₁₄Ester und 7,8 Gew.-% C₂₀-Diester.

### Beispiel 8

### Co-Metathese von 10-Undecensäuremethylester mit 4-Octen

Katalysator: WO₃-Re₂O₇ / Al₂O₃-SiO₂ (Al₂O₂-SiO₂ hydrothermal behandelt, 20 Gew.-% SiO₂) aktiviert mit Sn(n-C₄H₉)₄
Unter den Bedingungen von Beispiel 5, d.h. molares Verhältnis Re₂O₇: SnBu₄: Ester: 4-Octen = 1 : 1,5 : 1000 : 2000, jedoch mit einem WO₃-haltigen Katalysator steigt der Umsatz nach 3 Stunden bei Raumtemperatur auf 76 % und die Esterzusammensetzung betrug 22,0 Gew.-% C₁₁-Ester, 71,1 Gew.-% C₁₄-Ester und 6,9 Gew.-% Diester.
Die Herstellung des Katalysators erfolgte wie im Beispiel 1a beschrieben, jedoch wurden an Stelle von H₃BO₃ jetzt 1,59 g (NH₄)₁₀W₁₂O₄₁ eingesetzt. Der Katalysator enthielt 5,0 Gew.-% Re₂O₇ und 6,4 Gew.-% WO₃.

### Beispiel 9

### Co-Metathese von 10-Undensäuremethylester mit 4-Octen

Katalysator: MoO₃-Re₂O₇ / Al₂O₃-SiO₂ (Al₂O₃-SiO₂ hydrothermal behandelt, 20 Gew.-% SiO₂) aktiviert mit Sn(n-C₄H₉)₄
Unter den Bedingungen von Beispiel 5, d.h. molares Verhältnis Re₂O₇ : SnBu₄ : Ester : 4-Octen = 1 : 1,5 : 1000 : 2000, jedoch mit einem MoO₃-haltigen Katalysator steigt der Umsatz nach 3 Stunden bei Raumtemperatur auf 78 % und die Esterzusammensetzung betrug 18,9 Gew.-% C₁₁-Ester, 75,5 Gew.-% C₁₄-Ester und 5,4 Gew.-% Diester. Die Herstellung des Katalysators erfolgte wie im Beispiel 1a beschrieben, jedoch wurden an Stelle von H₃BO₃ jetzt 1,77 g (NH₄)₆Mo₇O₂₄ eingesetzt. Der Katalysator enthielt 5,0 Gew.-% Re₂O₇ und 6,4 Gew.-% MoO₃.

### Beispiel 10

### Co-Metathese von 10-Undecensäuremethylester mit 4-Octen

Katalysator: V₂O₅-Re₂O₇ / Al₂O₃-SiO₂ (Al₂O₃-SiO₂ hydrothermal behandelt, 20 Gew.-% SiO₂) aktiviert mit Sn (n-C₄H₉)₄.
Unter den Bedingungen von Beispiel 5, d.h. molares Verhältnis Re₂O₇ : SnBu₄ : Ester : 4-Octen = 1 : 1,5 : 1000 : 2000, jedoch mit einem V₂O₅-haltigen Katalysator steigt der Umsatz nach 5 Stunden bei Raumtemperatur auf 77 % und die Esterzusammensetzung betrug 20,2 Gew.-% C₁₁-Ester, 74,2 Gew.-% C₁₄ - Ester und 5,6 Gew.-% Diester. Die Herstellung des Katalysators erfolgte wie im Beispiel 1 beschrieben, jedoch wurden an Stelle von H₃BO₃ jetzt 0,968 g NaVO₃ eingesetzt. Der Katalysator enthielt 5,0 Gew.-% Re₂O₇ und 6,4 Gew.-% V₂O₅.

### Beispiel 11

### Co-Metathese von 10-Undecensäuremethylester mit 4-Octen, Versuche zum Katalysatorrecycling

Katalysator: B₂0₃ - Re₂0₇ /Al₂0₃ - SiO₂ (Al₂O₃-SiO₂ hydrothermal behandelt), aktiviert mit Sn(n-C₄H₉)₄
Bei der Katalysatorherstellung nach Beispiel 1 wurden zwei Parameter variiert. Zu einem wurde das hydrothermal behandelte Aluminiumsilikat mit 20 Gew.-% SiO₂ in Extrudatform verwendet, zum anderen wurden 10 g calciniertes Trägermaterial nur mit 0,11 g Ammoniumperrhenat bei gleichbleibender Menge Borsaure (1,33 g) versetzt, so daß ein Katalysator mit nominell 1 Gew.-% Re₂0₇ resultierte.
Dieser Katalysator wurde in die Co-Metathese von 10-Undecensäuremethylester mit 4-Octen eingesetzt, nach Reaktionsende jeweils abfiltriert, bei 120 °C getrocknet und nach erneuter 12-stündiger Aktivierung bei 550 °C im Luftstrom bzw. anschließender SnBu₄-Zugabe wieder in obiger Metathesereaktion getestet.
Dabei wurde in allen Versuchen von folgendem Ansatz ausgegangen: 0,71 g Katalysator, 0,44 ml einer 0,1 molaren Zinntetrabutyllösung in Cyclohexan, 10 g 4-Octen und 8,8 g 10-Undecensäuremethylester. Die Reaktionsmischung wurde jeweils 6 Stunden bei Raumtemperatur in einem Laborrüttler geschüttelt. Das molare Verhältnis der Reaktionskomponenten betrug: Re₂0₇: SnBu₄: Ester: 4-Octen = 1: 1,5: 3000 : 6000.
Die Reaktivierung wurde 14 mal ohne größere Aktivitätsverluste des eingesetzten Kontakts durchgeführt. Die in den einzelnen Versuchen erzielten Umsätze sind in Tabelle 4 dargestellt.

**Tabelle 4**

| *Co-Metathese von 10-Undecensäuremethylester mit 4-Octen an B*_{*2*}*0*_{*3*} *-Re*_{*2*}*0*_{*7*} */ Al*_{*2*}*0*_{*3*}*-SiO*_{*2*}*, aktiviert mit Sn(n-C*_{*4*}*H*_{*9*}*)*_{*4*} *- Versuche zum Katalysatorrecycling -* | | | | | | | | |
|---|---|---|---|---|---|---|---|---|
| **Versuch** | 1/1 | 1/2 | 1/3 | 1/4 | 1/5 | 1/6 | 1/7 | 1/8 |
| **Reaktivierungscyklus** | frisch | 1 | 2 | 3 | 4 | 5 | 6 | 7 |
| **U [%]** | 72 | 74 | 75 | 69 | 71 | 70 | 70 | 66 |

| **Versuch** | 1/9 | 1/10 | 1/11 | 1/12 | 1/13 | 1/14 | 1/15 | |
|---|---|---|---|---|---|---|---|---|
| **Reaktivierungscyklus** | 8 | 9 | 10 | 11 | 12 | 13 | 14 | |
| **U [%]** | 65 | 64 | 60 | 56 | 64 | 61 | 60 | |

## Patentansprüche

1. Auf Aluminiumsilikaten geträgerte Rheniumoxid-Katalysatoren, **dadurch gekennzeichnet,** daß Aluminiumsilikat-Träger eingesetzt werden, die aus einer Silicium-Verbindung als SiO₂-Lieferanten und einer Aluminium-Verbindung in wäßrigem Milieu hergestellt sind, wobei das Aluminiumsilikat / Wasser Gemisch während und/oder nach bevorzugt nach der Reaktion der Silicium-Verbindung mit der Aluminium-Verbindung einer Temperaturbehandlung von 90 bis 235 °C vorzugsweise von 150 bis 200°C über einen Zeitraum von 0,5 bis 20 h vorzugsweise 1 bis 5 h unterzogen wird.

2. Katalysatoren gemäß Anspruch 1, **dadurch gekennzeichnet,** daß die Aluminiumsilikat-Träger 1,5 bis 50 Gew.-% SiO₂, vorzugsweise 5 bis 30 Gew.-% SiO₂ enthalten.

3. Katalysatoren gemäß einem der vorherigen Ansprüche, **dadurch gekennzeichnet,** daß der Rheniumoxid-Gehalt 0,5 bis 10 Gew.-%, vorzugsweise 1 bis 5 Gew.-% beträgt.

4. Katalysatoren gemäß einem der vorherigen Ansprüche, **dadurch gekennzeichnet,** daß die Katalysatoren Boroxid-, Wolframoxid-, Molybdänoxid- oder Vanadiumoxid- Zusätze enthalten.

5. Katalysatoren gemäß einem der vorherigen Ansprüche, **dadurch gekennzeichnet,** daß der Boroxid-, Wolframoxid-, Molybdänoxid- oder Vanadiumoxid-Gehalt 2 bis 10 Gew.-%, vorzugsweise 3 bis 8 Gew.-% beträgt.

6. Katalysatoren gemäß einem der vorherigen Ansprüche, **dadurch gekennzeichnet,** daß die Katalysatoren als Aktivatoren metallorganische Verbindungen, vorzugsweise Zinnalkyle enthalten.

7. Katalysatoren gemäß einem der vorherigen Ansprüche, **dadurch gekennzeichnet,** daß die Katalysatoren nach Gebrauch abgetrennt, getrocknet, zur Reaktivierung bei erhöhter Temperatur von 300 bis 700°C vorzugsweise bei 350 bis 550°C thermisch behandelt und wiederverwendet werden.

8. Verwendung der Katalysatoren gemäß einem der vorherigen Ansprüche für die Metathese von Olefinen und/oder funktionalisierten Olefinen.

9. Verwendung der Katalysatoren gemäß Anspruch 8 für die Metathese ungesättigter Carbonsäureester.

## Claims

1. Rhenium oxide catalysts carried on aluminium silicates, **characterised in that** aluminium silicate carriers made from a silicon compound as a SiO₂ supplier and an aluminium compound in aqueous environment are used, whereby the aluminium silicate/water mixture, during and/or after, preferably after, the reaction of the silicon compound with the aluminium compound, is subjected to a temperature treatment of 90 to 235°C, preferably of 150 to 200°C for a period of 0.5 to 20 hours, preferably for 1 to 5 hours.

2. Catalysts according to claim 1, **characterised in that** the aluminium silicate carriers contain 1.5 to 50% by weight SiO₂, preferably 5 to 30% by weight SiO₂.

3. Catalysts according to one of the preceding claims, **characterised in that** the rhenium oxide content is 0.5 to 10% by weight, preferably 1 to 5% by weight.

4. Catalysts according to one of the preceding claims, **characterised in that** the catalysts contain additions of boric oxides, tungsten oxides, molybdenum oxides or vanadium oxides.

5. Catalysts according to one of the preceding claims, **characterised in that** the content of boric oxide, tungsten oxide, molybdenum oxide or vanadium oxide is 2 to 10% by weight, preferably 3 to 8% by weight.

6. Catalysts according to one of the preceding claims, **characterised in that** the catalysts contain organometallic compounds as activators, preferably tin alkyls.

7. Catalysts according to one of the preceding claims, **characterised in that** the catalysts, after use, are separated, dried and then thermally treated for reactivation at elevated temperatures from 300 to 700°C, preferably at 350 to 550°C, and reused.

8. The use of the catalysts according to one of the preceding claims for the metathesis of olefins and/or functionalised olefins.

9. The use of the catalysts according to claim 8 for the metathesis of unsaturated carboxylic acid esters.

## Revendications

1. Catalyseurs d'oxyde de rhénium supportés par des aluminosilicates, caractérisés en ce que l'on utilise des supports d'aluminosilicates qui sont préparés en milieu aqueux à partir d'un composé de silicium comme fournisseur de SiO₂ et d'un composé d'aluminium, le mélange d'aluminosilicate et d'eau étant soumis, pendant et/ou après, de préférence après la réaction du composé de silicium avec le composé d'aluminium, à un traitement thermique à une température de 90 à 235°C, de préférence de 150 à 200°C, sur une période de temps de 0,5 à 20 heures, de préférence de 1 à 5 heures.

2. Catalyseurs selon la revendication 1, caractérisés en ce que les supports d'aluminosilicates contiennent 1,5 à 50% en poids de SiO₂, de préférence 5 à 30% en poids de SiO₂.

3. Catalyseurs selon l'une quelconque des revendications précédentes, caractérisés en ce que la teneur en oxyde de rhénium est de 0,5 à 10% en poids, de préférence de 1 à 5% en poids.

4. Catalyseurs selon l'une quelconque des revendications précédentes, caractérisés en ce que les catalyseurs contiennent des additions d'oxyde de bore, d'oxyde de tungstène, d'oxyde de molybdène ou d'oxyde de vanadium.

5. Catalyseurs selon l'une quelconque des revendications précédentes, caractérisés en ce que la teneur en oxyde de bore, en oxyde de tungstène, en oxyde de molybdène ou en oxyde de vanadium est de 2 à 10% en poids, de préférence de 3 à 8% en poids.

6. Catalyseurs selon l'une quelconque des revendications précédentes, caractérisés en ce que les catalyseurs contiennent comme activateurs des composés organométalliques, de préférence des étain-alkyles.

7. Catalyseurs selon l'une quelconque des revendications précédentes, caractérisés en ce que les catalyseurs sont séparés après usage, séchés, traités par voie thermique pour les réactiver à une température élevée de 300 à 700°C, de préférence de 350 à 550°C, et réutilisés.

8. Utilisation des catalyseurs selon l'une quelconque des revendications précédentes pour la métathèse d'oléfines et/ou d'oléfines fonctionnalisées.

9. Utilisation des catalyseurs selon la revendication 8 pour la métathèse d'esters d'acides carboxyliques insaturés.
